# EUROPEAN PATENT SPECIFICATION

(11) **EP 2 886 150 B1**
(45) Date of publication and mention of the grant of the patent: **16.10.2019**
(21) Application number: 14004320.9
(22) Date of filing: 19.12.2014
(51) Int. Cl.: A61M 25/06

(54) **Jugular catether**
Jugularkatether
Cathéter jugulaire

(30) Priority: 20.12.2013 IT MU20130041
(43) Date of publication of application: 24.06.2015
(73) Proprietor: Delta Med S.p.A., 46019 Viadana (Mantova) (IT)
(72) Inventor: Brugioni, Lucio, 41121 MODENA (IT)
(74) Representative: Bergamini, Silvio

(56) References cited:
- WO-A1-00/64527
- WO-A1-01/70324
- WO-A1-2010/075445
- WO-A2-2005/035043
- US-A1- 2012 136 320
- US-A1- 2013 079 746
- US-B1- 7 186 222

## Description

### Field of the invention

This invention relates to a catheter system with a catheter, which can be generally used to reach the internal jugular vein of a patient, for administration of substances and sampling, without requiring invasive placement procedures.

### Background art

Central venous catheterization is known to be performed on patient for bolus or continuous administration of drugs, or for hydration or parenteral nutrition of patients.

In practice, a venous catheter is introduced into the patient, to reach a central vein that can provide a large-bore venous access for administration of considerable flows of drugs in various forms or for patient nutrition or hydration.

The venous catheter must be inserted in a surgery unit by a skilled physician and with the presence of an anesthetist-resuscitator, because the venous catheter typically has a considerable length and may extend to the heart chambers.

Therefore, prevention of possibly life-threatening side effects, such as pneumothorax or malignant arrhythmias, is required.

Furthermore, although placement of the venous catheter is guided, the patient must later undergo radiological examination to confirm that the venous catheter has been correctly placed and hence is in the desired position for therapy.

According to US2012/0136320 "methods of transvascular retrograde access placement and devices for facilitating the placement" is known.

The system has a handle, a catheter extending from the handle and a tissue piercing element extending from the handle through the catheter.

The tissue piercing element has a sharp distal tip adapted to extend from a distal portion of the catheter.

The catheter and the handle are adapted to be separated from the tissue piercing element which has a distal portion adapted to serve as a guide for introduction of a device into the central blood vessel.

According to WO2010/075445 "Methods and systems for treatment of acute ischemic stroke" are known.

According to this patent application, methods and devices are disclosed that enable safe, rapid and relatively short and straight access to the cerebral arteries for the introduction of interventional devices to treat acute ischemic stroke.

In addition, the disclosed methods and devices provide means to securely close the access site to the central arteries to avoid the potentially devastating consequences of a transcervical hematoma.

The above described prior art suffers from certain drawbacks.

A first drawback is that the placement of a venous catheter in a central vein is particularly difficult and may present dangers for patient safety.

A second drawback is that the placement of a venous catheter in a central vessel of a patient requires transfer of the latter to an operating room, as well as the manual and technical skills of an experienced physician to insert the catheter, and the presence of an anesthetist.

A third drawback is that the prior art involves high costs aa it requires some organization, with the presence of two physicians for safe placement of the venous catheter.

### Disclosure of the invention

One object of the invention is to improve the prior art.

Another object of the invention is to provide a jugular catheter that can reach a large-bore central vein, namely the jugular vein, without transferring the patient to an operating room.

A further object of the invention is to provide a jugular catheter that does not require the presence of an anesthetist-resuscitator for placement in a central vein, namely the jugular vein.

Yet another object of the invention is to provide a jugular catheter that can afford an improved technique for insertion and placement thereof into a central vessel of a patient, i.e. the jugular vein, thereby considerably reducing safety hazards for the latter.

The invention provides a catheter system with a jugular catheter as defined by the features of claim 1.

The invention achieves the following advantages:
- reaching the jugular vein of a patient and having large-bore access for administration and sampling;
- placing the jugular catheter in controlled and guided fashion inside the jugular vein;
- avoiding transfer of the patient to an operating room, as the venous catheter may be inserted while the patient is in bed;
- reducing the hazards of insertion of a venous catheter into a central vein.

### Brief description of the drawings

Further features and advantages of the invention will be more readily apparent upon reading of the detailed description of a preferred non exclusive embodiment of a jugular catheter, which is shown as a non limiting example by the annexed drawings, in which:
FIG. 1 is a bottom view of the interior of the front portion of the neck of a patient, namely under the jaw bone, in which the circulatory system is generally shown, and in which a jugular catheter of the invention is inserted;
FIG. 2 is a full-scale view of a jugular catheter and the introducer needle of the invention in an extracted position.

### Detailed description of a preferred embodiment

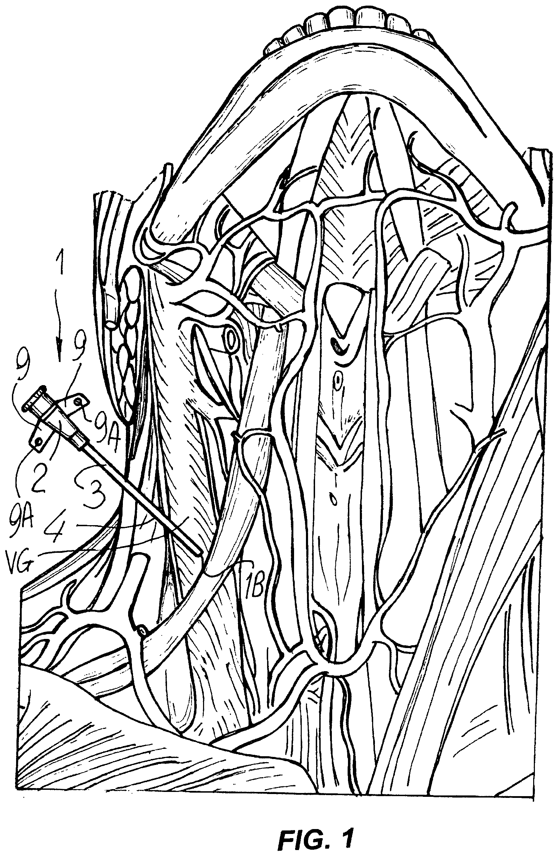

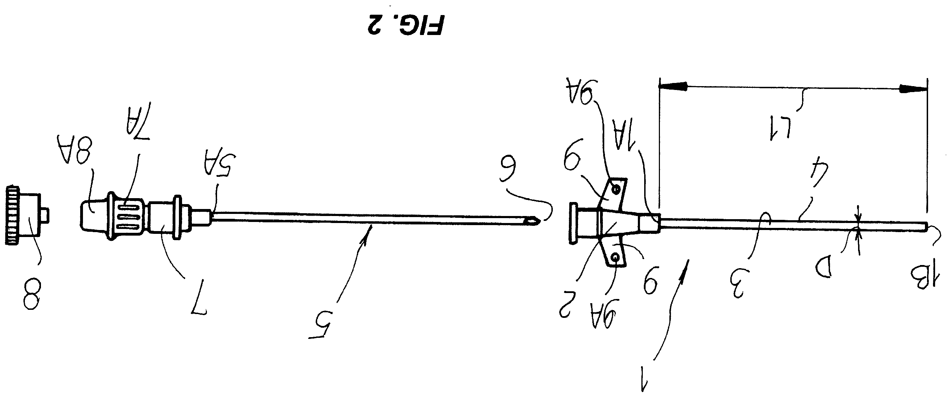

Referring to the above mentioned figures, numeral 1 generally designates a jugular catheter comprising a support element 2, typically known as "hub", which is associated with a distal end 1A of a flexible shaft 4 having an axial cavity 3, and a free and open distal end 1B.
For the particular use of the jugular catheter 1, which is designed to be introduced into the jugular vein of a patient, referenced "VG" in Figure 1, through the neck, the length of the shaft 4, referenced "L1" in Figure 2, shall range from 5.5 cm to 10 cm, whereas the diameter "D" shall range from 12 to 18 Gauge, which means that in the metric system the diameter "D" may range from 1.2 mm to 2.8 mm.

As shown in Figure 2, the jugular catheter 1 is in a shorter form, an introducer needle 5 being used for insertion and placement thereof in the jugular vein "VG", whose diameter is smaller than that of the axial cavity 3 and whose length is slightly greater than the length "L1" such that, when the introducer needle 5 is inserted in the axial cavity 3, in a ready-for-introduction state, the tip 6 of the introducer needle 5 slightly projects out of the distal end 1B of the shaft 4.

The introducer needle 5 also has a support element 7, which is fixedly associated with the proximal end 5A facing away from the tip 6.

The support element 7 is also axially hollow and is equipped with a removable closing cap 8, which is adapted to be pressed on a standard conical mouth 8A of the support element 7, facing away from the tip 6.

An additional element 7A is interposed between the support element 7 and the closing cap 8, and accommodates a filter whose purpose is to prevent backflow of blood from the vessel of the patient, while maintaining a passage for air.

The support element 2 of the jugular catheter 1 is also equipped with a pair of bilaterally outwardly extending flexible wings 9 which are designed to allow fixation of the jugular catheter to the epidermis of the patient by means of an adhesive strap or tape or a suture, the wings 9 having through holes 9A for passage of the latter.

It shall be noted that, in a possible embodiment, the jugular catheter 1 may be equipped with elements designed to be detected by detection equipment, such as ultrasound or X-ray imaging equipment.

Alternatively, the catheter 1 may be simply made with a plastic material having pigments added therein, for detection by the above mentioned detection equipment.

The operation of the jugular device 1 is as follows.

A physician holds the jugular catheter 1 in its ready-for-insertion state, i.e. with the introducer needle 5 inserted in the axial cavity 3.

Once the physician has determined the insertion area on one side of the neck of a patient, he/she punctures it with the needle tip and reaches the jugular vein "VG", introduces the end 1B and part of the shaft 4 therein, and continues inserting the jugular catheter 1 until the support element 2 abuts the epidermis of the patient.

Since the physician knows the length "L1" of the shaft 4 beforehand, he/she can determine when the support element 2 will abut the epidermis of the neck, and at the same time the end 1B of the shaft 4 will be correctly placed in the jugular vein "VG" for a length that is suitable for the therapy to be performed, without requiring the patient to undergo any X-ray examination for confirmation.

Furthermore, the length "L1" of the shaft 4 prevents the shaft to cause pneumothorax reactions or malignant arrhythmias in the patient.

Once the placement of the jugular catheter 1 has been completed, the physician pulls back the introducer needle 5 therefrom and has free direct access to the jugular vein "VG" of the patient for infusional therapy.

It shall be noted that, when the jugular catheter 1 is required to be equipped with detectable elements, the shaft 4 is simply made of a material with externally detectable pigments embedded therein, or a radiopaque material, and in both cases is visible by usual detection equipment: this allows confirmation of proper placement in the jugular vein "VG", when required, using the above mentioned detection equipment.

The invention has been found to fulfill the intended objects.

The invention so conceived is susceptible to changes and variants within the inventive concept.

Also, all the details may be replaced by other technical equivalent elements.

In its practical implementation, any material, shape and size may be used as needed, without departure from the scope as defined by the following claims.

## Claims

1. A catheter system comprising a jugular catheter (1), the jugular catheter (1) comprises:
- a flexible, axially hollow shaft (4) having a proximal end (1A) and an opposite distal end (1B);
- a support element (2), which is fixedly associated with said proximal end (1A), has a passage (3) substantially coaxial with said shaft (4)
**characterised in that** the catheter system further comprises a positioning needle (5) adapted to extend through the passage (3) in the shaft (4) and wherein said positioning needle (5) has a tip (6), slightly projecting out of the distal end (1B) of the shaft (4) when the needle is inserted into the passage (3) of the shaft (4) in a ready-for introduction state, and configured to be introduced in the jugular vein through the neck of a patient, and wherein the positioning needle (5) further comprises a support element (7) equipped with an additional element (7A) wherein a filter avoiding back flows is accommodated, and **in that** said shaft (4) of the jugular catheter (1) has both:
- a calibrated length (L1) ranging from 5.5 cm to 10 cm and an outside diameter ranging from 1,2 mm to 2,8 mm (12 Gauge to 18 Gauge)

2. A catheter system as claimed in claim 1, wherein said shaft comprises externally detectable detection means.

3. A catheter system as claimed in claim 2, wherein said detection means comprise means that can be detected by ultrasound and/or X-ray imaging equipment.

4. A catheter system as claimed in claim 2, wherein said detection means are selected from pigments embedded in said shaft or radiopaque materials used to form said shaft.

## Patentansprüche

1. Kathetersystem, umfassend einen Jugularkatheter (1), wobei der Jugularkatheter (1) umfasst
- einen flexiblen, axial hohlen Schaft (4), der ein proximales Ende (1A) und ein entgegengesetztes distales Ende (1B) aufweist;
- ein Trägerelement (2), das fest dem proximalen Ende (1A) zugeordnet ist und einen Kanal (3) aufweist, der im Wesentlichen zum Schaft (4) koaxial ist,
**dadurch gekennzeichnet, dass** das Kathetersystem weiter eine Positionierungsnadel (5) umfasst, die angepasst ist, um sich durch den Kanal (3) in den Schaft (4) zu erstrecken, und wobei die Positionierungsnadel (5) eine Spitze (6) aufweist, die geringfügig aus dem distalen Ende (1B) des Schafts (4) vorsteht, wenn die Nadel in einem für die Einführung bereiten Zustand in den Kanal (3) des Schafts (4) eingeführt ist, und die ausgelegt ist, um durch den Hals eines Patienten in die Jugularvene eingeführt zu werden, und wobei die Positionierungsnadel (5) weiter ein Trägerelement (7) umfasst, das mit einem zusätzlichen Element (7A) ausgestattet ist, wobei ein Filter, der Rückfluss verhindert, untergebracht ist, und dadurch, dass der Schaft (4) des Jugularkatheters (1) beides aufweist:
- eine kalibrierte Länge (L1) im Bereich von 5,5 cm bis 10 cm und einen Außendurchmesser im Bereich von 1,2 mm bis 2,8 mm (12 Gauge bis 18 Gauge).

2. Kathetersystem nach Anspruch 1, wobei der Schaft extern erkennbare Erkennungsmittel umfasst.

3. Kathetersystem nach Anspruch 2, wobei die Erkennungsmittel Mittel umfassen, die mittels bildgebenden Ultraschall- und/oder Röntgengeräten erkannt werden können.

4. Kathetersystem nach Anspruch 2, wobei die Erkennungsmittel ausgewählt sind aus in den Schaft eingebetteten Pigmenten oder strahlenundurchlässigen Materialien, die verwendet werden, um den Schaft zu bilden.

## Revendications

1. Système de cathéter comprenant un cathéter jugulaire (1), le cathéter jugulaire (1) comprend :
- un arbre flexible axialement creux (4) ayant une extrémité proximale (1A) et une extrémité distale opposée (1B) ;
- un élément de support (2), qui est associé de manière fixe à ladite extrémité proximale (1A), présente un passage (3) sensiblement coaxial audit arbre (4) ;
**caractérisé en ce que** le système de cathéter comprend en outre une aiguille de positionnement (5) adaptée pour s'étendre à travers le passage (3) dans l'arbre (4) et dans lequel ladite aiguille de positionnement (5) présente une pointe (6), légèrement en saillie à l'extérieur de l'extrémité distale (1B) de l'arbre (4) lorsque l'aiguille est insérée dans le passage (3) de l'arbre (4) dans un état prêt pour une introduction, et
configuré pour être introduit dans la veine jugulaire à travers le cou d'un patient, et dans lequel l'aiguille de positionnement (5) comprend en outre
un élément de support (7) muni d'un élément supplémentaire (7A), dans lequel un filtre évitant des reflux est reçu, et **en ce que** ledit arbre (4) du cathéter jugulaire (1) présente à la fois :
- une longueur calibrée (L1) dans la plage de 5,5 cm à 10 cm et un diamètre extérieur dans la plage de 1,2 mm à 2,8 mm (calibre 12 à calibre 18).

2. Système de cathéter selon la revendication 1, dans lequel ledit arbre comprend des moyens de détection détectables de manière externe.

3. Système de cathéter selon la revendication 2, dans lequel lesdits moyens de détection comprennent des moyens qui peuvent être détectés par un équipement d'imagerie par ultrasons et/ou par rayons X.

4. Système de cathéter selon la revendication 2, dans lequel lesdits moyens de détection sont sélectionnés à partir de pigments incorporés dans ledit arbre ou à partir de matériaux radio-opaques utilisés pour former ledit arbre.
